# EUROPEAN PATENT APPLICATION

(11) **EP 3 372 257 A1**
(43) Date of publication of application: **12.09.2018**
(21) Application number: 17160169.3
(22) Date of filing: 09.03.2017
(51) Int. Cl.: A61M 1/00

(54) **NEGATIVE PRESSURE WOUND THERAPY ASSEMBLY**

(71) Applicant: Mölnlycke Health Care AB, 415 02 Göteborg (SE)
(72) Inventor: NORTHWOOD, Ewen, Leeds, LS17 9HE (GB)
(74) Representative: Kransell & Wennborg KB

(57) **Abstract**

The present disclosure relates to a negative pressure wound therapy assembly (1, 100, 200, 300, 400) comprising a wound dressing (2). The wound dressing (2) comprises a cover layer (3) having a proximal side (4) adapted to face at least a first and a second wound tissue site (18a,18b) when in use and a distal side (5), opposite to the proximal side (4). The cover layer (3) comprises at least one separation line (14) dividing the cover layer (3) in at least a first and a second cover layer section (3a,3b), the at least first and second cover layer sections (3a,3b) extending side-by-side. The first cover layer section (3a) comprises a first cover layer interior portion (13a) and a first cover layer attachment portion (7a) enclosing the first cover layer interior portion (13a), and the second cover layer section (3b) comprises a second cover layer interior portion (13b) and a second cover layer attachment portion (7b) enclosing the second cover layer interior portion (13b), wherein at least a portion of each of the cover layer attachment portions (7a,7b) comprises fastening means on the proximal side (4) of the cover layer (3). The wound dressing furthermore comprises a first and second negative pressure application zone (15a,15b), and the first cover layer interior portion (13a) covers the first negative pressure application zone (15a) and the second cover layer interior portion (13b) covers the second negative pressure application zone (15b). The first and second negative pressure application zones (15a, 15b) are adapted to provide negative pressure to the first and second wound tissue sites (18a,18b) respectively

## Description

### TECHNICAL FIELD

The present disclosure relates to a negative pressure wound therapy assembly. More specifically, the present disclosure relates to a negative pressure wound therapy assembly especially suitable for use in multiple access surgical incision sites.

### BACKGROUND

Some types of wounds are advantageously treated by so called negative pressure wound therapy. In the field of negative pressure wound therapy, a negative pressure is applied to the wound for a relatively long time and it has been realized that the healing process may be expedited by using such wound therapy.

To this end, a negative pressure wound therapy system may be used which generally comprises a cover layer and an underlying manifold layer that is adapted to be placed over a wound. The system further generally comprises a negative pressure source, such as a vacuum pump, which is in fluid communication with the wound site via a suction port attached to the wound cover member and a fluid communication assembly comprising fluid conduit(s). The negative pressure source furthermore generally comprises a canister for collection of wound exudate removed from the wound site via the fluid communication assembly.

Negative pressure wound therapy dressings are conventionally supplied either as a set for single use, the set including a wound cover layer, a manifold layer and a suction port or as a single fully assembled wound dressing.

An increasing number of surgical procedures requires multiple incision sites and the need for multiple dressings and pumping systems is thereby increasing, resulting in increasing costs and complexity of the care.

### SUMMARY

One object of the present disclosure is to provide a negative pressure wound therapy assembly reducing costs for and/or facilitating the treatment of patients having a plurality of wound tissue sites to be treated.

This and other objects of the present disclosure may be achieved by a negative pressure wound therapy assembly according to claim 1.

As such, and in a first aspect, the present disclosure relates to a negative pressure wound therapy assembly. The negative pressure wound therapy assembly comprises a wound dressing comprising a cover layer having a proximal side adapted to face at least a first and a second wound tissue site when in use and a distal side, opposite to the proximal side. The cover layer comprises at least one separation line dividing the cover layer into at least a first and a second cover layer section, the first and second cover layer sections extending side-by-side. The first cover layer section comprises a first cover layer interior portion and a first cover layer attachment portion enclosing the first cover layer interior portion. The second cover layer section comprises a second cover layer interior portion and a second cover layer attachment portion enclosing the second cover layer interior portion. The cover layer attachment portions are each provided with fastening means on at least a portion thereof. The wound dressing furthermore comprises a first and a second negative pressure application zone, the first cover layer portion covering the first negative pressure application zone and the second cover layer portion covering the second negative pressure application zone. The first and the second negative pressure application zones are adapted to provide negative pressure to the at least first and second wound tissue sites respectively, during use.

The fact that the cover layer is provided with a separation line dividing the cover layer into at least a first and a second cover layer section facilitates a correct and predetermined separation of the first and a second cover layer section. By separating the cover layer into at least a first and a second cover layer section, a first and second wound dressing section is obtained which may be applied to separate wound tissue sites, e.g. located at a distance from each other.

The cover layer attachment portions are adapted to provide attachment of the cover layer sections to skin surrounding the wound tissue sites or to an underlying layer to provide a seal around the negative pressure application zones. In embodiments of the invention, the cover layer attachment portions may be provided with fastening means by providing a continuous fastening means layer, such as an adhesive layer, over the entire cover layer attachment portions respectively, or over the entire cover layer proximal side. In embodiments of the invention, the fastening means may be provided as a respective continuous frame surrounding each one of the first and second cover layer interior portions.

In embodiments of the invention, the fastening means is provided as a discontinuous adhesive layer enclosing each one of the cover layer interior portions, such as by means of a continuous pattern of dots and/or stripes enclosing and framing the cover layer interior portions. The entire proximal side of the cover layer may also be provided with the continuous adhesive pattern of for example dots and/or stripes.The adhesive layer should be arranged such that a tight seal to the skin or an underlying layer is provided.

In embodiments of the invention, the first cover layer section comprises a first circumferential edge portion and the first attachment portion comprises the circumferential edge portion, such that an attachment to an underlying surface may be provided over the entire first circumferential edge portion. The second cover layer section comprises a second circumferential edge portion and the second attachment portion comprises the second circumferential edge portion, such that an attachment to an underlying surface may be provided over the entire second circumferential edge portion.

The fact that attachment may be provided on the entire first and second circumferential edge portions respectively, according to the embodiments disclosed above, enables a secure sealing around the negative pressure zones and around the respective wound tissue site after separation of the cover layer along the separation line to a first and a second cover layer section.

The wound dressing may be provided with a protective release paper on the cover layer proximal side and over the negative pressure application zones to cover and protect the fastening means prior to use. The release paper may be any type of conventionally used release paper, such as a silicon or PE coated release paper.

By "negative pressure application zone" is meant a zone intended to face a wound tissue site and which zone is adapted apply and distribute negative pressure to the wound tissue sites, by providing a volume between the cover layer interior portion and the wound tissue site and permitting the transport of fluid (e.g. air and/or liquid) from the wound tissue site.

The at least first and second negative pressure application zones may each comprise a manifold layer facing the proximal side of the cover layer.

The manifold layers are thus provided to distribute negative pressure to the wound tissue sites and/or to drain or to deliver fluids therefore. The manifold layers may include a plurality of flow channels, which may distribute fluids provided to the wound tissue sites and/or which may remove the fluids therefrom.

In embodiments of the invention, the manifold layers are foam layers, a spacer fabrics or a combination thereof. The manifold layers may thus each comprise one layer or several interposed sublayers. The manifold layers may be provided in direct contact with the cover layer

By "spacer fabric" herein is meant a three-dimensional knitted or woven fabric consisting of two separate knitted or woven substrates which are interconnected by an interconnecting layer of filaments. The interconnecting layer of filaments may be a knitted polyester, viscose, cellulose or the like fibres, such as monofilament fibres. One example of such spacer layer is disclosed in WO 2011/144888.

In embodiments of the invention, the manifold layers comprise or consist of porous foam. The porous foam may be, for example, open-cell foam. Alternatively or additionally, exemplary manifold layers may comprise closed-cell foam that comprises through-holes. The through-holes may extend through the entirety of one dimension (e.g., the thickness) of the manifold layer. Such embodiments may allow fluid to flow through the manifold layer, and may be particularly suited for use in negative pressure wound treatment systems as described hereinbelow. Porous foams may be made of any suitable material, including, but not limited to, polymer foams as described herein. Non-limiting examples of suitable polymer foams include polyurethane foams, polyvinyl alcohol foams, silicone foams, polyolefin foams, alginate foams, and combinations thereof. In some embodiments, the polymer foam comprises polyurethane foam. Non-limiting examples of suitable polyurethane foams include polyester-based and polyether-based foams.

In embodiments of the invention, the manifold layers comprise or consist of a nonwoven material, e.g. spunbond, meltblown, carded, hydroentangled, wetlaid nonwovens etc. may be used. Suitable nonwoven materials can be composed of natural fibres, such as wood pulp or cotton fibres, manmade fibres, such as polyester, polyethylene, polypropylene, viscose etc., or from a mixture of natural and syntethic fibres.

By "wound tissue site" herein is meant an open or closed (e.g. closed incision) wound or damage located on or within any tissue, such as dermal tissue, bone tissue, adipose tissue, muscle tissue, neural tissue, vascular tissue, connective tissue, cartilage, tendons, or ligaments. The term "wound tissue site" may further refer to areas of any tissue that are not wounded or damaged, but are instead areas in which it is needs to promote the growth of tissue or stimulate the tissue.

By "separation line" is meant a continuous or discontinuous line, the line may be straight, curved or zig-zag shaped as long as it extends over the cover layer to provide a border separating the cover layer in a first and a second cover layer section.

In embodiments of the invention, the separation line comprises weakening means or separation indicium dividing the cover layer into at least a first and a second cover layer section. When the wound dressing is covered and protected by a protective release paper on the wound tissue site facing side, the release paper may also comprise a separation line corresponding in location to the cover layer weakening means or separation indicium.

In embodiments of the invention, the weakening means is a predefined line along which the strength of the cover layer is lower than the strength of the cover layer outside the predefined line, e.g. when a separation force is applied perpendicular to the cover layer, such as by pulling firmly, the cover layer is separated along the predefined line which become a single dividing line.

In embodiments of the invention, the weakening means is a series of discontinuous slits, perforations or embossings forming a line, or a line along which the cover layer has a lower thickness than outside the predefined line, allowing rupture of the cover layer at a predefined location.

In embodiments of the invention, the separation indicium is a printed or embossed continuous or discontinuous line. The separation indicium is intended to provide indication to the care givers about where to separate the cover layer sections, such as by cutting.

In embodiment of the invention, the negative pressure wound therapy assembly comprises at least a first and a second suction device, wherein the first suction device and the second suction device are attached or attachable to the first and second cover layer respectively, such that the first suction device is in fluid communication with the first negative pressure application zone and the second suction device is in fluid communication with the second negative pressure application zone and wherein the suction devices are adapted to be connected to a negative pressure source. The negative pressure source may be a common negative pressure source or a common canister.

In embodiments of the invention, the negative pressure source comprises a canister and a pump, wherein the canister is in fluid communication with the pump and wherein the canister is adapted to collect fluid from the wound tissue site. In some embodiments, the negative pressure source is canisterless.

The fluid communication between the suction devices and the negative pressure zones may be established via first and second openings provided in the first and second cover layer interior portions respectively.

The suctions devices function as suction interfaces adapted to connect the wound dressings to a negative pressure source, e.g. via a suction port and/or a conduit, and are each provided with a fluid inlet and a fluid outlet for application of a negative pressure to the wound tissue sites via the negative pressure application zone and to enable fluid removal from the wound tissue site.

In embodiments of the invention, the at least first and second suction devices include a first and a second suction port respectively and/or a first and a second fluid conduit respectively.

In embodiments of the invention, the at least first and second suction ports each comprises coupling means, and the first and second suction ports are adapted to be fluidly connected to the first and second fluid conduits by means of the coupling means. Optionally, the suction ports coupling means are adapted to form a push fit, snap fit, threaded, luer-lock or bayonet engagement with the fluid conduits. This provides for flexibility in the connections with the fluid conduits.

The type of conduits connecting the suction ports to the negative pressure source may be selected in accordance with the specific need.

In embodiments of the invention, the first and second suction devices comprise a first and a second suction port respectively, forming unitary components with a first and a second fluid conduit respectively, such as the suction portion and the fluid conduits being molded to form unitary components. The respective suction ports are thus accordingly each provided with a fluid conduit. This allows for an easy and quickly applied negative pressure wound therapy assembly with fewer steps during application and connection of the wound dressing sections after separation of the at least first and the second cover layer sections.

The provision of a negative pressure wound therapy assembly having the at least first and the second suction devices each comprising a suction port and a fluid conduit assembled and fluidly connected to the first and second negative pressure application zones, provides for an assembly which is cost efficient and easy to use in treatment of patients requiring simultaneous application of a plurality of negative pressure wound dressings, such as after surgical procedures requiring multiple incision sites, as it reduces the amount of separate wound dressings to handle, reduces entanglement of the conduits and also provides a possibility to connect several wound tissue sites to one negative pressure source.

The fluid conduits may for this reason be fluidly connected to a single fluid outlet which fluid outlet is adapted to be fluidly connected directly or indirectly to a negative pressure source. The fluid conduits may thus converge to a single fluid outlet, such as to a single fluid conduit with a single fluid outlet. The possibility to easily connect a plurality of negative pressure treated wound tissue sites to one single negative pressure source is both cost saving, convenient and less complex with a reduced number of conduits and negative pressure sources/canisters which may be caught and interfere with the patient treatment.

The fluid conduits may be separably connected to each other, such as in a longitudinal direction.

The fact that the fluid conduits are separably connected to each other allows for individual adaptation of the fluid conduits for each treatment occasion, such as adaptation due to the distance between the wound tissue sites to be treated.

In embodiments of the invention, the fluid conduits may be separably connected to each other by means of a separator clip.

In embodiments of the invention, the fluid conduits may be co-extruded to parallel fluid conduits separably connected to each other in a longitudinal direction.

In embodiments of the invention, each of the first and second suction ports may comprise an air feeding inlet.

The air feeding inlet allows for a supply of air through at least a portion of the suction port and into the fluid conduit allowing removal of fluids from the wound tissue site to provide flushing of the fluid removing conduits.

In embodiments of the invention, the suction ports may either be provided with air inlets provided with a bacterial filter directly at the suction port or alternatively each be provided with an additional air supplying conduit. In embodiments of the invention, the air inlets may be provided with filters adapted to control and thus provide a desired air flow therethrough.

The air supplying conduits may be provided to the suction ports in the same manner as the fluid conduits. Each of the fluid conduits may for example be co-extruded with an air supplying conduit and the first fluid conduit/air supplying conduit pair may be separably connected to the second fluid conduit/air supplying conduit pair.

In embodiments of invention, the first and second suction devices are attached or attachable to the distal side of the cover layer. For example, the first suction device may be attached on the distal side of the cover layer in the first cover layer interior portion and the second suction device may be attached on the distal side of the cover layer in the second cover layer interior portion.

In embodiments of the invention, the fluid communication between the suction devices and the negative pressure application zones are established by providing pre-made first and second openings in the first and second cover layer interior portions respectively and subsequently attaching the first and second suction devices over the respective first and second openings. In embodiments of the invention, a suction device which provides the cover layer with an opening when attaching the suction device to the cover layer may be applied, such as suction devices provided with aperture-forming elements. One non-limiting example of such suction device is illustrated in WO 2014/053232.

In embodiments of the invention, each of the suction ports comprises a suction port attachment portion, wherein the at least two suction devices are attached or attachable to the distal side of the cover layer by means of the respective attachment portions. The suction ports may be fixedly attached to the cover layer. In embodiments of the invention, the negative pressure wound therapy assembly is provided as a kit comprising the suction ports as separate components, each of the suction ports provided with an attachment portion, which are adapted to be attached, via the respective attachment portion, to the cover layer such that the first and the second suctions ports are in fluid communication with the first and the second negative pressure application zones respectively, such as via a first and second opening provided in the cover layer interior portions.

In embodiments of the invention, the first and the second suction devices may be arranged to extend on the proximal side of the cover layer. The first suction device extending from the first circumferential edge portion of the cover layer to the first negative pressure application zone and the second suction device extending from the second circumferential edge portion of the cover layer to the second negative pressure application zone. Meaning that, when the first and second wound dressing sections are attached to the skin or to an underlying layer by means of the first and second attachment portions respectively, and over a first and a second wound tissue site, the suction devices extend between the skin/an underlying layer and the edge of the cover layer and in to the interior portion being the negative pressure application zone, which zone may comprise a porous material allowing provision and distribution of a negative pressure provided via the suction devices. In such embodiments of the negative pressure wound therapy assembly the suction devices typically comprises fluid conduits.

An "underlying layer" may for example be a perforated plastic film layer (e.g. polyurethane or polypropylene) having a skin facing surface coated with an adhesive, such as a silicone gel based adhesive, wherein the perforated plastic film may be provided on the proximal side of the cover layer and extending over the fastening means and the negative pressure application zone to face the wound tissue site and the skin surrounding wound tissue site during use of the negative pressure wound therapy assembly. The perforations in the plastic film layer facilities fluid communication between the wound tissue site and the negative pressure application zone of the dressing through the perforation of the perforated film. In embodiments of the invention, the perforated plastic film comprises perforation only in a portion adapted to be positioned below negative pressure application zone. In embodiments of the invention, the "underlaying layer" may be a continuous plastic film layer having a skin facing surface coated with an adhesive wherein the plastic film layer comprises an opening adapted to be positioned below the negative pressure zone, thereby ensuring fluid communication between the wound tissue site and the negative pressure application zone of the dressing through the opening in the plastic film.

In embodiments of the invention, the cover layer may be a flexible plastic film, such as a polymeric film. The polymeric film may comprise a thermoplastic polymer, such as for example, a polyurethane, polyethylene, polypropylene, polyvinyl chloride, polystyrol, polyether, polyester, polyamide, polycarbonate, polyether polyamide copolymers, polyacrylate, polymethacrylate, and/or polymaleate. In embodiments of the invention, the cover layer is a polyurethane, polyolefin, polyester or polyamide film. The cover layer may be liquid impermeable. The cover layer may preferably have a low enough gas permeability to ensure that a desirable negative pressure can be maintained and to provide a barrier for bacteria, whilst, at the same time, the cover layer may still have some degree of water vapour permeability.

Furthermore, it is advantageous if the cover layer has a thickness in the area of from ≥ 10 µm up to ≤ 80 µm, particularly preferred of from ≥ 10 µm up to ≤ 60 µm, and/or that the cover layer has an elongation at break of more than 450%.

The fastening means may be an adhesive layer. Non-limiting examples on suitable adhesives are silicone based adhesive, hot-melts, or acrylic based adhesive.

### BRIEF DESCRIPTION OF THE DRAWINGS

The various aspects of the present disclosure, including its particular features and advantages will be readily understood from the following detailed description and the accompanying drawings, in which:
Fig. 1 a illustrates a cross-section of a negative pressure wound therapy assembly according to the present disclosure, as seen from the proximal side.
Fig. 1b illustrates a negative pressure wound therapy assembly according to Fig. 1a, as seen from the distal side.
Fig. 2 illustrates a negative pressure wound therapy assembly, as seen from the distal side.
Fig. 3 illustrates a cross-section of a negative pressure wound therapy assembly according to the present disclosure.
Fig. 4 illustrates a negative pressure wound therapy assembly according to the present disclosure, wherein the negative pressure wound therapy assembly sections has been separated into a first and a second negative pressure wound dressing section.
Fig. 5 illustrates a negative pressure wound therapy assembly according to the present disclosure.

### DETAILED DESCRIPTION

It is to be understood that the drawings are schematic and that individual components, such as layers of material and individual components are not necessarily drawn to scale. The negative pressure wound therapy assemblies shown in the figures are provided as examples only and should not be considered limiting to the invention.

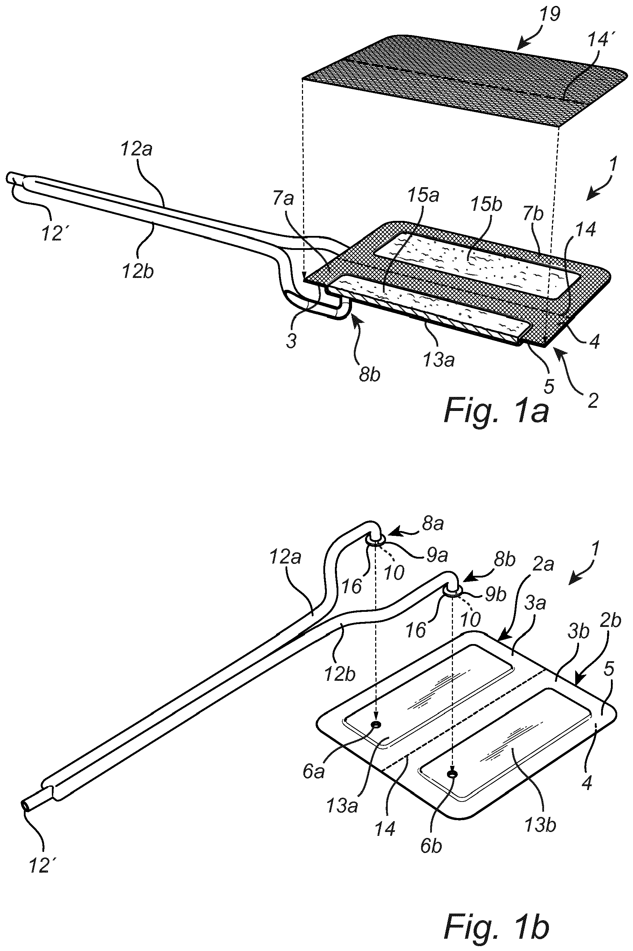

Figs. 1 a and 1 b illustrate a negative pressure wound therapy assembly 1 according to the present disclosure. The particular shape of the negative pressure wound therapy assembly should not be considered limiting to the invention. Accordingly, a negative pressure wound therapy assembly may have any useful shape or size.

Fig. 1 a illustrates the negative pressure wound therapy assembly 1 as seen from a wound tissue facing side, and Fig. 1b illustrates the negative pressure wound therapy assembly from Fig. 1a, as seen from a side adapted to face away from the wound tissue site during use.

The purpose of a negative pressure wound therapy assembly 1 is to apply a negative pressure in the area of a wound tissue site.

The negative pressure wound therapy assembly 1 illustrated in Figs. 1 a and 1 b comprises a wound dressing 2. The wound dressing 2 comprises a cover layer 3 in the form of a flexible plastic film having a proximal side 4 adapted to face a wound tissue site when in use and a distal side 5, opposite to the proximal side 4. The wound dressing 2 furthermore comprises a first and a second negative pressure application zone 15a, 15b, comprising a respective manifold layer facing the cover layer proximal side 4.

The cover layer 3 comprises a first and a second cover layer interior portion 13a,13b, enclosed by a first and a second attachment portion 7a,7b respectively. The first and second attachment portions 7a,7b are provided with fastening means on the proximal side 4 of the cover layer 3 in the form of an adhesive layer. The first attachment portion 7a is arranged on a first circumferential edge portion extending along the longitudinal and transverse outer edges of the first cover layer section 3a and the second attachment portion 7b is also arranged on a second circumferential edge portion extending along the longitudinal and transverse outer edges of the second cover layer section 3b. The circumferential edge portions have a width of at least 5 mm as measured from the cover layer outer edges.

In Fig. 1a, the adhesive layer is provided as a continuous layer on the proximal side 4 of the cover layer 3. The fastening means may of course be provided as a discontinuous layer, such as by means of a continuous pattern of dots and/or stripes provided over the attachment portions 7a,7b on the proximal side 4 of the cover layer 3, however if the adhesive layer is provided in a discontinuous pattern, the pattern needs to be arranged such that a tight seal to the skin or an underlying layer may be ensured.

The cover layer 3 comprises a first and a second opening 6a, 6b provided in the first and second cover layer interior portions 13a,13b, each extending from the proximal side 4 to the distal side 5 of the cover layer 3, as illustrated in Fig. 1 b.

The cover layer 3 is provided with a separation line 14 in the form weakening means provided as a series of discontinuous slits allowing rupture and separation of the cover layer 3 at a predefined location positioned such that the coextensive cover layer sections 3a and 3b positioned on each side of the weakening means may be separated from each other. According to Fig. 1b, the cover layer sections 3a and 3b are of equal size and are mirror-images of each other.

As illustrated in Fig. 1a, the proximal side 4 of the cover layer 3 and the manifold layers provided in the negative pressure application zones 15a,15b are covered with an aperture silicon coated film 19 providing the skin and wound tissue site contacting layer. Such a silicon coated film 19 may be coated with a release layer, providing protection. The silicon coated film 19 is provided with a separation line 14' in the form of weakening means corresponding in location with the separation line 14 provided in the cover layer 3.

The negative pressure wound therapy assembly 1 is provided with a first and second suction device 8a, 8b. The first suction device comprises a first suction port 9a and a first fluid conduit 12a and the second suction device 8b comprises a second suction port 9b and a second fluid conduit 12b. The first suction port 9a is attached to the cover layer 3 in the first cover layer interior portion 13a at the distal side 5 and over the first opening 6a. The second suction port 9b is attached to the cover layer 3 in the second cover layer interior portion 13b at the distal side 5 and over the second opening 6b. The first and second suction port 9a, 9b each comprises a fluid inlet 10 and are each provided with suction port attachment portions 16 for facilitating attachment to the cover layer 3. The first and second suction devices 8a, 8b are in fluid communication with the respective first and second negative pressure application zone 15a,15b via the respective first and second opening 6a,6b in the cover layer 3 such that a negative pressure may be applied to the wound tissue sites and wound tissue exudate may be transported from the wound tissue sites.

The first and second suction ports 9a, 9b form unitary components with the first and the second fluid conduit 12a,12b respectively, which are thus fluidly connected to the first and second suction port 9a, 9b.

The first and the second conduit 12a,12b are separably connected to each other in a longitudinal direction and converge to a single fluid outlet 12'. The single fluid outlet 12' may be adapted to connect directly to a negative pressure source, or may also be adapted to be connected indirectly to a negative pressure source, such as to an intermediate connection system. The fact that the conduits 12a,12b converge into one single conduit having one fluid outlet 12' reduces the complexity with a plurality of conduits which may be caught and interfere with the patient treatment and cost as only one negative pressure source is needed.

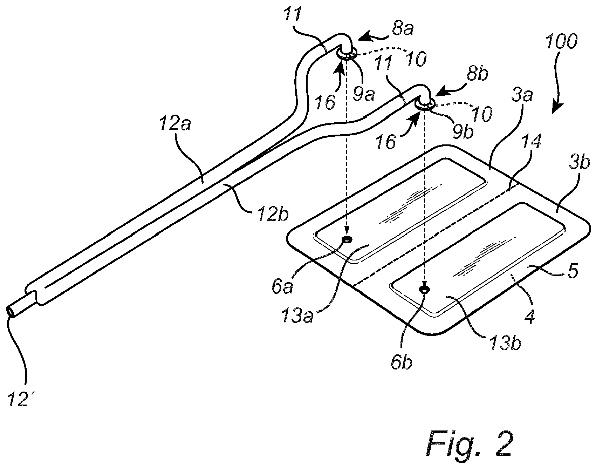

Figure 2 illustrates a negative pressure wound therapy assembly 100 according to the present disclosure as seen from the distal side 5.

The first and second suction devices 8a,8b comprises a first and a second suction port 9a,9b which are attached or attachable to the cover layer 3 at the distal side 5, by means of the suction port attachment portions 16, over the first and second openings 6a, 6b, respectively. The first suction port 9a is connected to a first conduit 12a and the second suction port 9b is connected to a second fluid conduit 12b by means of coupling means 11 provided at the first and second fluid ports 9a,9b.

The cover layer 3 is furthermore provided with a separation line 14, in the form of discontinuous slits allowing rupture of the cover layer 3 at a predefined location and allowing the first and the second cover layer sections 3a,3b to be fully separated from each other. The separation line 14 may of course alternatively be in the form of separation indicia, such as a printed or embossed line. The first and second cover layer section 3a,3b illustrated in Fig. 2 have essentially the same size. However, these may of course also be specifically adapted to a specific surgical procedure and constitute of, for example, one larger section and one smaller section.

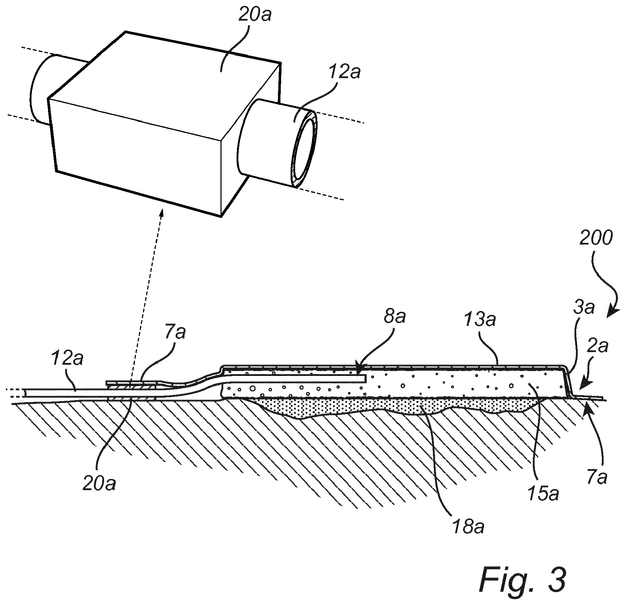

Fig. 3 illustrates a cross section of a negative pressure wound therapy assembly 200 according to the present disclosure. The first wound dressing section 2a, and the first negative pressure application zone 15a is applied over a first wound tissue site 18a and the cover layer attachment portion 7a is attached to the skin surrounding the first wound tissue site 18a. The suction device 8a comprises a fluid conduit 12a extending under the cover layer 3, from the circumferential edge portion of the cover layer 3 to the negative pressure application zone 15a, which is provided as a foam layer. The fluid conduit 12a is provided with an attachment layer 20a to provide attachment to the attachment portion 7a of the cover layer 3, and to ensure that the fluid conduit 12a is maintained in a fixed position.

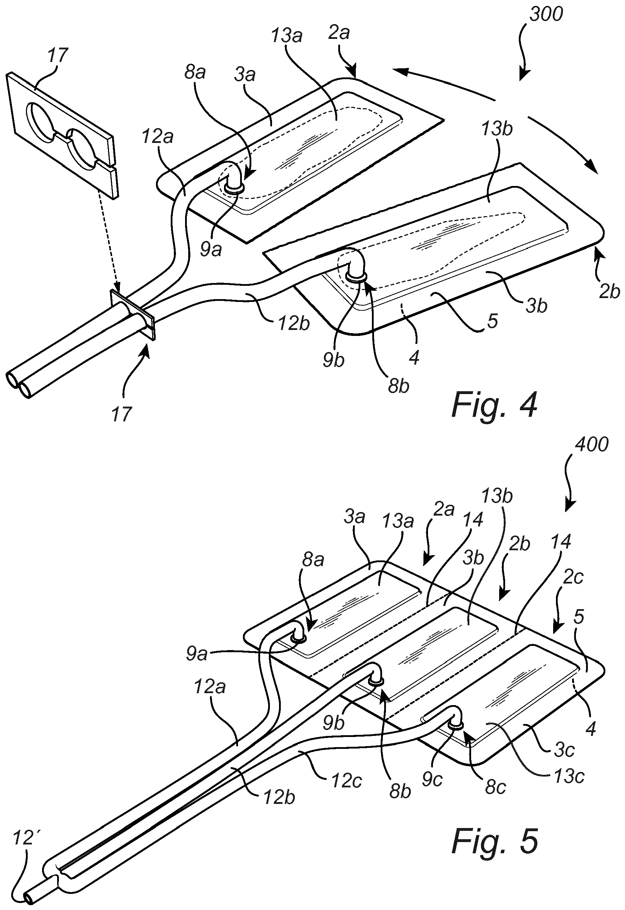

Fig. 4 illustrates the negative pressure wound therapy assembly 300 after the first and second cover layer sections 3a,3b have been pulled apart at the separation line 14 and are fully separated from each other, thus providing a first and a second separated wound dressing section 2a,2b. The first and second negative pressure applications zones (not shown) are applied over a first and a second wound tissue site 18a,18b respectively. The conduits 12a,12b are separably connected to each other by means of a separator clip 17 allowing adjustment of the connection between the conduits 12a,12b.

Fig. 5 illustrates a negative pressure wound therapy assembly 1 according to the present disclosure comprising a wound dressing, including a first, second and third wound dressing section 2a,2b,2c. The wound dressing comprises a cover layer, divided in a first, second and third cover layer section 3a,3b,3c by means of separation lines 14, in the form of discontinuous slits allowing rupture of the cover layer at a predefined location and allowing the first, the second and the third cover layer sections 3a,3b,3c to be fully separated from each other in use by firmly pulling the sections apart in a direction essentially perpendicular to the travelling direction of the separation lines 14. The cover layer is furthermore provided with a first, second and a third opening (not shown) in each of the respective first, second and third cover layer interior portions 13a, 13b, 13c. A first, second and third suction device 8a,8b,8c, each comprising a suction port 9a,9b,9c and a conduit 12a, 12b, 12c, are attached to the cover layer 3 at the distal side 5 and over the first, second and third openings respectively. The first, second and third conduits 12a,12b,12c converge to a single fluid outlet 12'.

The wound dressing furthermore comprises a first, second and third negative pressure application zone, each in the form of a foam layer, facing the cover layer proximal side 4 and being in fluid communication with the first, second and third openings respectively.

## Claims

1. A negative pressure wound therapy assembly (1, 100, 200, 300, 400) comprising a wound dressing (2), said wound dressing (2) comprising a cover layer (3) having a proximal side (4) adapted to face at least a first and a second wound tissue site (18a,18b) when in use and a distal side (5), opposite to said proximal side (4), said cover layer (3) comprising at least one separation line (14) dividing said cover layer (3) in at least a first and a second cover layer section (3a,3b), said at least first and second cover layer sections (3a,3b) extending side-by-side, wherein said first cover layer section (3a) comprises a first cover layer interior portion (13a) and a first cover layer attachment portion (7a) enclosing said first cover layer interior portion (13a), wherein said second cover layer section (3b) comprises a second cover layer interior portion (13b) and a second cover layer attachment portion (7b) enclosing said second cover layer interior portion (13b), wherein at least a portion of said cover layer attachment portions (7a,7b) each comprises fastening means on said proximal side (4) of said cover layer (3), said wound dressing furthermore comprising a first and second negative pressure application zone (15a,15b), wherein said first cover layer interior portion (13a) cover said first negative pressure application zone (15a) and said second cover layer interior portion (13b) cover said second negative pressure application zone (15b), said first and second negative pressure application zones (15a, 15b) being adapted to provide negative pressure to said first and second wound tissue sites (18a,18b) respectively.

2. The negative pressure wound therapy assembly (1, 100, 200, 300, 400) according to claim 1, wherein said first cover layer section (3a) comprises a first circumferential edge portion, wherein said first attachment portion (7a) comprises said first circumferential edge portion and wherein said second cover layer section (3b) comprises a second circumferential edge portion, wherein said second attachment portion (7b) comprises said second circumferential edge portion, such that attachments to an underlying surface is enabled along the entire of said first and said second circumferential edge portions respectively.

3. The negative pressure wound therapy assembly (1, 100, 200, 300, 400) according to claims 1 or 2, wherein each one of said first and second negative pressure application zones (15a,15b) comprises a manifold layer facing said proximal side (4) of said cover layer (3).

4. The negative pressure wound therapy assembly (1, 100, 200, 300, 400) according to claim 3, wherein each of said manifold layers comprises a foam layer and/or a spacer fabric.

5. The negative pressure wound therapy assembly (1, 100, 200, 300, 400) according to any of the preceding claims, wherein said separation line (14) comprises weakening means or separation indicium.

6. The negative pressure wound therapy assembly (1, 100, 200, 300, 400) according to claim 5, wherein said weakening means is a series of discontinuous slits, perforations, allowing rupture of said cover layer (3) at a predefined location.

7. The negative pressure wound therapy assembly (1, 100, 200, 300, 400) according to claim 5, wherein said separation indicium is a printed or embossed continuous or discontinuous line.

8. The negative pressure wound therapy assembly (1, 100, 200, 300, 400) according to any of the preceding claims, wherein said negative pressure wound therapy assembly (1) comprises at least a first and a second suction device (8a,8b), said first and second suction devices (8a,8b) being attached or attachable to said cover layer (3) such that said first suction device (8a) is in fluid communication with said first negative pressure application zone (15a) and said second suction device (8b) is in fluid communication with said second negative pressure application zone (15b), said suction devices (8a,8b) being adapted to be in fluid communication with a negative pressure source.

9. The negative pressure wound therapy assembly (1, 100, 200, 300, 400) according to claim 8, wherein said first and second suction devices (8a,8b) comprises a first and a second suction port (9a,9b) respectively, and a first and a second fluid conduit (12a,12b), respectively.

10. The negative pressure wound therapy assembly (1, 100, 200, 300, 400) according to claim 9, wherein said fluid conduits (12a,12b) are fluidly connected to a single fluid outlet (12'), which fluid outlet (12') is adapted to be connected to a negative pressure source.

11. The negative pressure wound therapy assembly (1, 100, 200, 300, 400) according to claims 9 or 10, wherein said fluid conduits (12a,12b) are separably connected to each other.

12. The negative pressure wound therapy assembly (1, 100, 300, 400) according to any one of claims 8-11, wherein said first and said second suction devices (8a,8b) are attached or attachable to said cover layer (3) on said distal side (5).

13. The negative pressure wound therapy assembly (1, 100, 300, 400) according to claim 12, wherein said first and second cover layer interior portions (13a,13b) are provided with a respective first and second opening (6a,6b).

14. The negative pressure wound therapy assembly (200) according to any one of claims 8-11, wherein said first and said second suction devices (8a,8b) are arranged to extend, on said proximal side (4) of said cover layer (3), from said first and said second circumferential edge portion, respectively, to said first and said second negative application zones (15a,15b), respectively.

15. The negative pressure wound therapy assembly (1, 100, 200, 300, 400) according to any one of the preceding claims, wherein said fastening means is an adhesive layer.
